# EUROPEAN PATENT APPLICATION

(11) **EP 4 846 941 A1**
(43) Date of publication of application: **02.12.2026**
(21) Application number: 26181869.4
(22) Date of filing: 29.05.2026
(51) Int. Cl.: A61B 6/00

(54) **MEDICAL IMAGING SCANNING METHOD, SYSTEM, COMPUTER DEVICE, AND STORAGE MEDIUM**

(30) Priority: 30.05.2025 CN 202510726145
(71) Applicant: Shanghai United Imaging Healthcare Co., Ltd., Shanghai 201801 (CN)
(72) Inventor: DIAO, Xuanhang, Shanghai, 201801 (CN); WANG, Xu, Shanghai, 201801 (CN); WU, Yefen, Shanghai, 201801 (CN); SHA, Liuli, Shanghai, 201801 (CN)
(74) Representative: Herrero & Asociados, S.L.

(57) **Abstract**

A medical imaging scanning method, a system, a computer device, and a storage medium. The method includes: in response to a localization instruction for a to-be-scanned object, acquiring (S110) a localization image of the to-be-scanned object, and performing (S120) a localization verification on the localization image; when a localization verification result fails to meet a first condition, displaying (S130) first prompt information to prompt repositioning of the to-be-scanned object until the localization verification result meets the first condition; at a position corresponding to a result of the localization verification meeting the first condition, performing (S130) an image scanning to obtain a scan image; performing a scan verification on the scan image, and when a scan verification result fails to meet a second condition, displaying (S140) second prompt information to prompt rescanning the to-be-scanned object until at least one new scan image meeting the second condition is obtained.

## Description

### TECHNICAL FIELD

The present disclosure relates to the field of medical technologies, and in particular, to a medical imaging scanning method, a system, and a computer-readable storage medium.

### BACKGROUND

In current medical imaging workflows, numerous algorithms are adopted for assistance. These algorithms may significantly improve imaging scanning efficiency. However, each algorithm has its adapted input data distribution. When the input data does not conform to the preset data distribution assumed during algorithm design, the correctness of the algorithm cannot be guaranteed, thereby affecting the correctness of subsequent operations and ultimately leading to deviations in the scanning results.

### SUMMARY OF THE DISCLOSURE

Based on the above, it is necessary to provide a medical imaging scanning method, a system, a computer device, and a computer-readable storage medium, as set out in the appended set of claims, to address the technical problem that the aforementioned methods are prone to causing deviations in scanning results.

In the aforementioned medical imaging scanning method, system, computer device, and storage medium, after localizing the to-be-scanned object is completed, the localization image is acquired and analyzed. Moreover, when the result of the localization verification does not meet the first condition, first prompt information is displayed to indicate inaccurate localization, requiring re-localization. This ensures accurate localization of the to-be-scanned object, thereby making the localization of the subsequently acquired scan image appropriate, and only the quality of the scan image needs to be verified subsequently. During scan image verification, verification is performed according to the requirements for acquiring the scan image. When the result of the scan verification does not meet the second condition, second prompt information is displayed to indicate that the scan image quality does not meet the requirements, prompting rescanning, such that the finally output scan image meets the requirements of subsequent processing. This may ensure the reliability of input data for each step of the medical imaging scanning workflow, improves scanning quality, and can reduce manual workload, enhancing scanning efficiency.

### BRIEF DESCRIPTION OF THE DRAWINGS

FIG. 1 is a flowchart of a medical imaging scanning method according to some embodiments of the present disclosure.
FIG. 2 is a flowchart of operations for localization image verification according to some embodiments of the present disclosure.
FIG. 3 is a flowchart of acquisition, verification, and interaction of a localization image according to some embodiments of the present disclosure.
FIG. 4 is a flowchart of operations for scan verification according to some embodiments of the present disclosure.
FIG. 5 is a flowchart of acquisition, verification, and interaction of a scan image according to some embodiments of the present disclosure.
FIG. 6 is a flowchart of a medical imaging scanning method according to some other embodiments of the present disclosure.
FIG. 7 is a schematic diagram of a workflow for automatically calculating TI time in an application example.
FIG. 8 is a flowchart of acquisition, verification, and interaction of TI Scout images according to some embodiments of the present disclosure.
FIG. 9 is a flowchart of calculating TI time from TI Scout images according to some embodiments of the present disclosure.
FIG. 10 is a schematic diagram of a fully automated PSIR workflow with full-process quality control according to some embodiments of the present disclosure.
FIG. 11 is a structural block diagram of a medical imaging scanning system according to some embodiments of the present disclosure.
FIG. 12 is an internal structural diagram of a computer device according to some embodiments of the present disclosure.

### DETAILED DESCRIPTION

To make the objectives, technical solutions, and advantages of the present disclosure clearer, the present disclosure is described in further detail below with reference to the accompanying drawings and embodiments. It should be understood that the specific embodiments described herein are merely for explaining the present disclosure and are not intended to limit it.

It should be noted that the terms "first", "second", and the like in the description and claims of the present disclosure and the above-mentioned drawings are intended to distinguish similar objects and are not necessarily to describe a specific order or sequence. It should be understood that data used in this way may be interchanged under appropriate circumstances, such that the embodiments of the present disclosure described herein may be implemented in an order other than those illustrated or described.

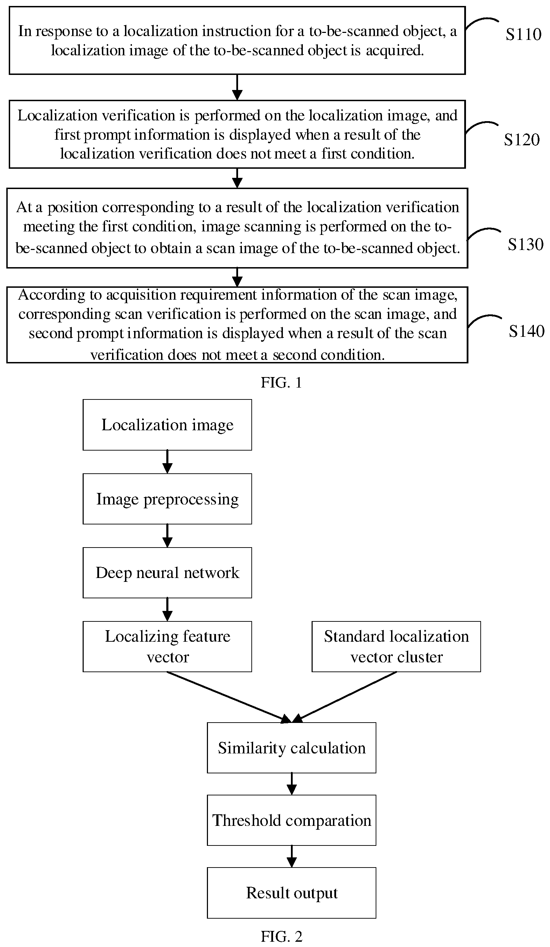

In some embodiments, as shown in FIG. 1, a medical imaging scanning method is provided. The embodiments are described with the method being applied to a terminal as an example. It can be understood that the method may otherwise be applied to a server, or to a system including a terminal and a server, and implemented through interaction between the terminal and the server. The terminal may be, but is not limited to, various personal computers, laptops, smartphones, tablets, Internet of Things (IoT) devices, and portable wearable devices. The IoT devices may be smart speakers, smart TVs, smart air conditioners, smart vehicle-mounted devices, etc. The portable wearable devices may be smart watches, smart bracelets, head-mounted devices, etc. The server may be implemented as a standalone server or a server cluster consisting of multiple servers. In some embodiments, the method includes the following operations.

At block S110: In response to a localization instruction for a to-be-scanned object, a localization image of the to-be-scanned object is acquired.

The to-be-scanned object may be a part of a human body, such as the head, neck, heart, abdomen, liver, kidney, stomach, leg, etc.; or the to-be-scanned object may be the whole human body.

The localization is configured to select a suitable scanning position for subsequent imaging.

The localization image may be a preliminary image configured to confirm the scanning position for the to-be-scanned object. Through analysis of the localization image, it may be confirmed whether the current localization is appropriate and accurate. The localization image may be obtained by scanning by a medical imaging equipment or by capturing by a camera. It is understood that when the localization image is from a camera, the standard localization image cluster, which will be discussed in the following, may include typical optical images. For example, the medical imaging equipment may include a digital radiography (DR) device, an x-ray imaging (XR) device, a computed tomography (CT) device (e.g., a Fan Beam computed tomography (FBCT) device, a cone beam computed tomography (CBCT) device), a positron emission tomography (PET) device, a magnetic resonance imaging (MRI) device, a medical ultrasonography device, a positron emission tomography/computed tomography (PET/CT) device, a positron emission tomography/magnetic resonance imaging (PET/MRI) device, or the like, or any combination thereof.

In some embodiments, the localization instruction may be initiated by a user or automatically. In some embodiments, the localization of the to-be-scanned object may be performed by the user. After the localization, the localization image of the to-be-scanned object may be acquired. A localization verification operation may be triggered by the user or automatically. In some embodiments, the localization of the to-be-scanned object may be performed automatically. After the localization, the localization image of the to-be-scanned object may be acquired. A localization verification operation may be triggered by the user or automatically.

In some embodiments, the triggering of the localization instruction may be done in two ways. One is manual localization, performed by the user. When the user finishes manual localization adjustment (for example, the user clicks a "confirm" control, or any operation indicating the end of slice selection, such as releasing the left mouse button which is for dragging a localization box), a localization instruction is generated, triggering a localization verification operation. The other is automatic localization. When the localization is finished via an automatic localization algorithm, a localization instruction is generated, triggering the localization verification operation.

At block S120: Localization verification is performed on the localization image, and first prompt information is displayed when a result of the localization verification does not meet a first condition; where the first prompt information is configured to prompt to perform re-localization of the to-be-scanned object until the result of the localization verification meets the first condition.

The first condition is configured to determine whether the localization verification passes, e.g., based on image characteristics of the localization image.

In some embodiments, a standard localization image cluster may be set. Verification of the localization image may be performed based on the standard localization image cluster. When the result of the localization verification of the localization image fails to meet the first condition, it indicates that the current localization does not meet the requirements and needs adjustment. Therefore, by displaying the first prompt information, the user is prompted to readjust a scanning localization of the to-be-scanned object. This process continues until the result of the localization verification of the newly obtained localization image meets the first condition, and the position of the to-be-scanned object at this time is taken as a position for subsequent image scanning. In some embodiments, the requirements or the first condition may include an image quality of the localization image meets a clinical demand, the localization of the to-be-scanned object meets a clinical demand, or the like, or any combination thereof. For example, when there is no artifact in the localization image or the artifact in the localization image is within an acceptable range, the image quality of the localization image meets the clinical demand. As another example, when the to-be-scanned object is within a field of view (FOV) of the medical imaging equipment, the localization of the to-be-scanned object meets the clinical demand. In some embodiments, the requirements or the first condition herein may involve subjective criteria-namely, whether the localization image is clear and appropriate-or objective assessments across at least one of three dimensions: modality, semantics, and pathology, which will be discussed later.

At block S130: At a position corresponding to a result of the localization verification meeting the first condition, image scanning is performed on the to-be-scanned object to obtain a scan image of the to-be-scanned object.

The scan image may be understood as actual image data obtained by scanning the to-be-scanned object, for subsequent medical analysis and diagnosis.

In some embodiments, after obtaining the localization where the result of the localization verification meets the first condition, image scanning may be further performed on the to-be-scanned object at the position corresponding to this localization to obtain the scan image. For example, magnetic resonance scanning is performed to obtain magnetic resonance images. As another example, CT (Computed Tomography) scanning is performed to obtain CT images.

At block S140: According to acquisition requirement information of the scan image, a scan verification is performed on the scan image, and second prompt information is displayed when a result of the scan verification does not meet a second condition; where the second prompt information is configured to prompt rescanning the to-be-scanned object until obtaining a scan image meeting the second condition.

Scan verification is configured to evaluate the scan image to determine whether it meets requirements for subsequent analysis/diagnosis.

The second condition may include that an image quality of the at least one scan image meets a clinical demand for subsequent medical analysis and diagnosis. The second condition is a standard for judging quality of the scan image, ensuring that image data is sufficient for subsequent medical analysis/diagnosis.

In some embodiments, the acquisition requirement information may be understood as the requirements for subsequent processing (e.g., the subsequent analysis/diagnosis) of the acquired scan image. Different acquisition requirement information corresponds to different verification requirements for the scan image. Therefore, when verifying the scan image after obtaining the scan image, the verification of the scan image may be performed according to the actual requirements, which may be reflected by the acquisition requirement information, also the requirements for subsequent processing, of acquiring the scan image. Correspondingly, the second condition is also determined according to the acquisition requirement information of the scan image, such that the scan image that passes the scan verification meets the requirements of subsequent processing. When the result of the scan verification does not meet the second condition, that is, the obtained scan image does not meet the requirements of subsequent processing, the second prompt information is displayed to prompt rescanning the to-be-scanned object, for obtaining a new scan image and performing scan verification, until a newly obtained scan image having a result of the scan verification that meets the second condition is obtained, which may facilitate subsequent image processing and analysis.

In some embodiments, the at least one scan image may share the same second condition. In some alternative embodiments, the second condition may be different for different scan images. For illustration purposes, it should be noted that after acquiring the localization image for localization verification, the subsequent acquired scan images may be one or multiple, that is, not limited to the second condition in the embodiments, and there may be a third condition, a fourth condition, etc., for different scan images.

In the above medical imaging scanning method, after localizing the to-be-scanned object is completed, the localization image is acquired and analyzed. Moreover, when the result of the localization verification does not meet the first condition, the first prompt information is displayed to indicate inaccurate localization, requiring re-localization. This may ensure accurate localization of the to-be-scanned object, thereby making the localization of the subsequently acquired scan image appropriate, and only the quality of the scan image is required to be verified subsequently. During scan image verification, verification is performed according to the requirements for acquiring the scan image. When the result of the scan verification does not meet the second condition, the second prompt information is displayed to indicate that the scan image quality does not meet the requirements, prompting rescanning, such that the finally output scan image meets the requirements of subsequent processing. This may ensure the reliability of input data for each step of the medical imaging scanning workflow, improve scanning quality, and reduce manual workload, thereby enhancing scanning efficiency.

In some embodiments, the localization instruction is an orientation determination instruction for the to-be-scanned object during scanning; in the aforementioned operation S110, the performing localization verification on the obtained localization image includes: determining a standard localization image cluster based on a type of the to-be-scanned object, where the type includes an orientation (e.g., patient positioning) corresponding to the localization instruction or a body part corresponding to the to-be-scanned object; and verifying the localization image based on the standard localization image cluster to obtain the result of the localization verification.

In some embodiments, the type of the to-be-scanned object may be understood as the type of body part, such as heart, brain, etc. In some embodiments, the type of the to-be-scanned object may refer to at least one of a shape of the to-be-scanned object, an age of the to-be-scanned object, a weight of the to-be-scanned object, the location and orientation of the to-be-scanned object, or clinical requirements.

The standard localization image cluster may include multiple standard localization images. The standard localization images may adopt images acquired from objects during correct localization operations of the relevant workflow. Moreover, the standard localization images are not limited to images of a single object, and localization images from multiple different objects may be collected to reduce the impact of individual differences on subsequent result of the localization verifications, thereby improving the reliability of the result of the localization verifications.

Scanning a same part may have multiple orientation types. For example, the TI Scout scan orientation may be cardiac short-axis, two-chamber, or four-chamber view. In some embodiments, each orientation type for each part may be built as an image cluster. Candidate localization image clusters may be pre-built for multiple orientation types of multiple parts. A standard localization image cluster matching the type of the to-be-scanned object and the orientation corresponding to the localization instruction may be selected from the candidate localization image clusters. Then, the similarity between the current localization image and each standard localization image in the selected standard localization image cluster may be calculated to determine the result of the localization verification. The similarity calculation method may be vector distance measurement methods such as Euclidean distance, Manhattan distance, cosine distance, Mahalanobis distance, and their variants.

In some embodiments, a first feature extraction model may be adopted to perform feature extraction on the localization image and each standard localization image respectively, and then the similarity between the feature vector of the localization image and the feature vector of each standard localization image may be obtained. In some embodiments, the first feature extraction model may be a neural network (e.g., a deep neural network) or a model built based on feature engineering methods such as sparse representation. The first feature extraction model is obtained by training on sample data. When the first feature extraction model is a neural network, the sample data may include positive sample data and negative sample data. The positive sample data represents sample localization images that meet requirements (which may be reflected by the acquisition requirement information, also the requirements for subsequent processing), and the negative sample data represents sample localization images that do not meet requirements. In some embodiments, since there are various body parts as to-be-scanned objects, such as the head, heart, etc., to improve the accuracy of verification results, corresponding feature extraction models may be trained for different body parts. For example, a head feature extraction model is trained using sample localization images of the head for feature extraction of head localization images. In another example, a heart feature extraction model is trained using sample localization images of the heart for feature extraction of heart localization images.

In some embodiments, the feature vector of each standard localization image may be extracted in advance via the first feature extraction model, and these feature vectors corresponding to the standard localization images are saved as a standard localization vector cluster. After acquiring the localization image, only the feature vector of the localization image is required to be extracted, and then similarity is directly calculated with the feature vectors of each standard localization image.

It is understandable that the standard localization image cluster may be updated from time to time. Users may independently add custom standard localization images or delete existing standard localization images that do not meet their requirements based on their own experience and preferences, making the verification of localization images more adaptable to the user's scanning habits.

In some embodiments, before performing the localization verification on the obtained localization image, the localization image may be preprocessed to improve the quality of the localization image. Further verification may be performed on the preprocessed localization image. The preprocessed localization image is input into the first feature extraction model to obtain a feature vector. The similarity between the feature vector and each standard localization vector in the standard localization vector cluster is calculated, and the similarity is compared with a threshold to determine whether the localization image meets the requirements. When the localization image meets the requirements, it indicates accurate localization. This process is shown in FIG. 2.

The preprocessing of the localization image may include the following methods: (1) Pre-annotating the region of interest (ROI) through a model. For example, the model may be an object detection model, a segmentation model, a dedicated model for corresponding images, or a general large model. Segmentation accuracy may be pixel (or voxel)-level precise segmentation and annotation, or may be coarse detection or segmentation that only provides anchor boxes or larger areas. The above methods themselves, their variants, and combinations thereof may further improve the accuracy of the quality control task. (2) Uniform cropping or interpolation to a uniform size; (3) Windowing, suppressing high-brightness pixels (or voxels), and then performing min-max normalization of voxel (or pixel) values in each image to the range of 0~1. Direct normalization may be performed, where only pixel (or voxel) values within a certain percentile range are operated on to suppress high-brightness pixels (or voxels). For example, only values within the range of the minimum value and the 0.9 quantile are subjected to min-max normalization to the range of 0~1. It may be understood that the localization image may be 2D or 3D.

In some embodiments, by combining the type of the to-be-scanned object and the corresponding localization, standard localization image clusters may be customized for various different scanning scenarios, thereby making the method more adaptable, significantly enhancing the accuracy, adaptability, and efficiency of the medical imaging scanning process.

In addition to the aforementioned quality control method based on standard image clusters and feature vector similarity calculation, the present disclosure further provides a semantic quality control method based on a Visual Language Model (VLM). This method may be used independently or combined with the aforementioned quality control method based on standard image clusters to form an integrated quality control solution. For example, the localization verification is performed via the aforementioned quality control method based on standard image clusters and feature vector similarity calculation. As another example, the localization verification is performed via the VLM. As a further example, the localization verification is performed via the aforementioned quality control method based on standard image clusters and feature vector similarity calculation in combination with the VLM.

In some embodiments, the visual language model quality control module includes the following components.

Input component, configured to receive a medical image to be detected (e.g., the localization image or the scan image of the to-be-scanned object), and optionally, one or more preset prompt templates and contextual information (such as examination type, patient information, etc.).

Processing component, including a visual encoder for converting the input image into a visual feature representation; an optional language encoder for processing prompts; an optional multimodal fusion module for integrating visual and linguistic features; and an output generation module for generating a quality assessment report in natural language form.

Output component, configured to output a structured quality assessment report. This report may include a quality score (e.g., 1-5 points or pass/fail), abnormal type identification (e.g., motion artifact, metal artifact, orientation error, etc.), abnormal location localization (describing the area where the anomaly is located), and/or a natural language explanation (stating the judgment basis and suggestions).

The visual language model may include, but is not limited to: general-purpose large multimodal models (such as GPT-4V, Qwen-VL, etc.), medical-specific visual language models (such as Med-PaLM M, LLaVA-Med, etc.), or privately hosted lightweight models (fine-tuned on private data based on open-source models like CLIP, LLaVA, etc.).

To guide the model for professional quality control, a preset prompt template may be adopted. For example, for localization image quality control, the prompt may be designed as: "You are a senior MRI imaging technician, checking the quality of a localization image. Examination: {exam_type}, Target organ: {target_organ}. Please analyze the following aspects and output in a structured format: 1. Quality score; 2. Positioning assessment; 3. Artifact detection; 4. Scan coverage; 5. Comprehensive suggestions; 6. Rationale".

When the feature vector method is used in combination with the visual language model method, the system may include a fusion decision module. This module processes according to the following logic.
1. Consistent Scenario: When both the methods determine as "pass" or "fail", a consistent conclusion is output, and the detailed explanation generated by the visual language model may be attached.
2. Conflict Scenario: When the determinations of the two methods are inconsistent, a prompt indicating "requires manual review" is output. Meanwhile, the determination basis of each method (e.g., the similarity score/heatmap from the feature vector method, and the quality report generated by the large model) is displayed for the user's reference in decision-making.
3. Confidence Weighting (Optional): The system may assign different confidence weights to the two methods based on their historical performance data, and make a final decision by calculating a weighted composite score, thereby improving the reliability of the overall decision.

In some embodiments, the first condition includes that a deviation degree of the localization image relative to the standard localization image cluster does not exceed a first threshold, or, the deviation degree exceeds the first threshold but a user's acceptance instruction is received; the method further includes: when the result of the localization verification does not meet the first condition, displaying warning information, and/or the deviation degree of the localization image relative to the standard localization image cluster, and taking the displayed warning information and/or the deviation degree as the first prompt information.

Forms of the warning information may include but are not limited to pop-up windows, interface icons, text prompts, localization box highlighting, etc.

In some embodiments, when the result of the localization verification does not meet the first condition, meaning it does not meet the localization requirements for subsequent scanning, warning information and/or the deviation degree of the localization image relative to the standard localization image cluster may be displayed as the first prompt information. Specifically, when displaying the warning information, the user may be allowed to view the relevant images and decide whether to continue scanning. Displaying the deviation degree may be shown while prompting that the localization image does not meet requirements. Specifically, a difference between an average similarity between the current localization image and each standard localization image and the first threshold may be displayed. When the average value is only slightly greater than the first threshold, the user may be prompted to manually intervene and check (e.g., the user decides whether to proceed to the next step). When the average value is far greater than the first threshold, the user may be prompted, and the previous operations may be repeated directly without requesting user permission.

In some embodiments, the user's determination criteria are related to actual requirements. For example: In the localization for a sagittal scan based on a head positioning image, when the localization verification module determines that the localization of the midsagittal plane of the head is deviated (e.g., the tissue morphology in the slice differs significantly from a normal slice), which would affect the reading/interpretation effect for areas like the pituitary gland and the midline brain structures, the user for such examinations may not accept the positioning result. However, when the area of interest (to-be-scanned object) in an examination does not require high precision for the sagittal plane orientation, such as in examinations of the cerebellum, etc., the user (e.g., MR technician) may determine that this deviation has little impact on performing subsequent scanning and may accept the position corresponding to the localization image.

In some embodiments, a new first threshold may be determined in response to a user's modification operation, making the new first threshold more consistent with the actual situation of the current device's operation.

It is understandable that, generally, the deviation degree of an image relative to a standard image includes deviations in one or more of the following three dimensions: modality deviation, semantic deviation, and pathologic deviation. The modality deviation may be determined based on: (1) Whether the image is of an expected modality (including whether the image contrast, style are consistent, and whether key imaging parameters are consistent or similar); (2) Whether the image illustrates a target body part; (3) Whether the image illustrates a target orientation. The semantic deviation typically refers to the imaging effect of the target area and may be determined based on: (1) Whether focusing on the target organ or part, while ignoring non-target areas; (2) Whether there are severe artifacts and noise in the target area. The pathology deviation typically refers to abnormal brightness in the target area due to lesions. Generally, modality deviation > semantic deviation > pathology deviation.

Herein, the target body part may refer to a specific human anatomical structure or organ intended to be observed, diagnosed, or analyzed within a medical imaging scan plan. For example, in a cardiac magnetic resonance examination, the target body part could be the "left ventricular myocardium"; in a cranial magnetic resonance examination, the target body part could be the "pituitary gland". The target body part defines the spatial objective of the scan, i.e., "what to scan".

The target orientation may refer to a spatial posture of the patient's body relative to the imaging equipment, along with a pre-defined geometric plane (section) of the scan, to optimally visualize the anatomical structures and pathological features of the target body part. For instance, for the cardiac region, common "intended scanning orientations" include the cardiac short-axis view, two-chamber view, and four-chamber view. The target orientation defines the geometric perspective of the scan, i.e., "from which direction to scan".

In the embodiments, the present disclosure categorizes deviations of an image relative to a standard image into three hierarchical levels: "modality deviation", "semantic deviation", and "pathological deviation", and establishes the general relationship that "modality deviation degree > semantic deviation degree > pathological deviation degree". This categorization is based on the fundamental impact that problems of different natures have on whether the image can serve its intended purpose.

Herein, the modality deviation refers to errors in the fundamental attributes or acquisition conditions of the image, causing the image to fail the basic premises of the scanning protocol. Examples include acquiring an image of an unexpected modality (e.g., obtaining a T2-weighted image when a T1-weighted image was needed), scanning the wrong anatomical site (e.g., scanning the liver when the heart was intended), or a severe error in orientation/plane (e.g., not using the short-axis view for a cardiac scan). Such deviations render the image entirely unusable for the intended workflow. Therefore, the degree of the modality deviation may be judged as the highest.

The semantic deviation refers to issues with the imaging quality of the image, under the premise that the modality is correct, affecting the clear observation and subsequent analysis of the target body part. The semantic deviation primarily includes diagnostic-significant severe artifacts (e.g., motion artifact, metal artifact), excessive noise, and/or improper scan coverage leading to incomplete display of the target. Such deviations compromise the usability of the image and the reliability of analytical results and are the primary focus of quality control. Therefore, the degree of the semantic deviation may be considered secondary to the modality deviation.

The pathological deviation refers to features manifested in the image due to the patient's own disease or physiological abnormality, such as abnormal signals caused by tumors, inflammation, or infarct foci, which is the target information that medical imaging aims to discover, not a defect. Therefore, within the quality control workflow, it is necessary to distinguish such "true positive" findings from "false positive" abnormal signals caused by quality issues. An effective quality control method shall be able to tolerate or ignore pathological manifestations. Therefore, the degree of the pathological deviation may be judged as the lowest.

In some embodiments, the role of the localization image is preliminary localization, so the baseline for its verification may consider only the image modality. Therefore, the first threshold may be set to a value less than a modality deviation value and greater than a semantic deviation value, such that during verification, there are only two cases: "with modality deviation" and "without modality deviation". Correspondingly, the deviation degree of the localization image relative to the standard localization image cluster is the deviation degree in image modality. The modality deviation value may be obtained by calculating an average distance of historical localization images with modality deviation relative to the standard localization image cluster. The semantic deviation value may be obtained by calculating an average distance of historical localization images with semantic deviation relative to the standard localization image cluster.

In the embodiments, the first condition is set as the deviation degree of the localization image from the standard localization image cluster not exceeding the first threshold, to ensure scanning accuracy to the greatest extent. In addition, it is set that even if the deviation degree exceeds the first threshold, the user may be still allowed to choose to accept it under the premise of fully understanding the risks. This flexibility may adapt to different clinical requirements, avoiding unnecessary repeated scans due to overly strict judgment standards, thereby improving scanning efficiency. Furthermore, considering that during localization verification, incorrect slice localization may lead to a large degree of abnormality, the method of issuing warnings and numerical prompts of the deviation degree is adopted as prompt information to ensure the reliability of the prompt information.

In the verification method based on feature vector similarity, the "deviation degree" may be quantified by calculating the distance between the feature vector of a current image and each of the feature vectors in the corresponding standard image cluster, and then taking the average of these distances.

The determination of "slightly greater than" and "significantly greater than" a threshold may be precisely defined by setting dual thresholds: Let a preset acceptable threshold be t1, and another higher threshold be t2 (t1 < t2); then: when the average value < t1, the current image is determined as meeting the requirements (normal); when t1 ≤ average value < t2, the current image is determined as "slightly greater than" the threshold, and the system prompts that manual review is required, allowing the user to decide whether to proceed to the next stage; when the average value ≥ t2, the current image is determined as "significantly greater than" the threshold, and the system may directly trigger the repetition of previous steps (such as re-positioning or re-scanning, i.e., the dual-threshold scheme in the embodiments may be applicable to the verification of scan images) while prompting the user, without waiting for user permission.

The settings of thresholds t1 and t2 may be determined based on manual experience or engineering experiments, or may be dynamically calculated based on the statistical characteristics of the standard image cluster (such as the distribution's covariance matrix, Mahalanobis distance, etc.).

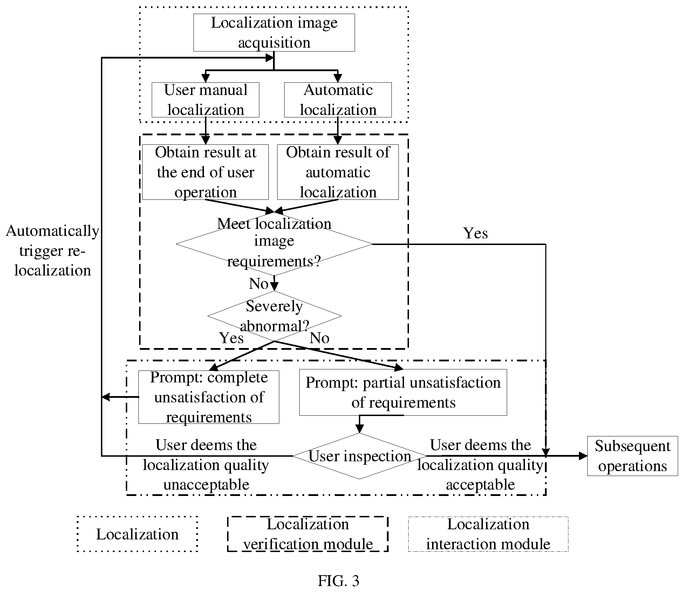

To facilitate understanding of the acquisition, verification, and interaction of the localization image in the above embodiments, further explanation is provided below in conjunction with the schematic flowchart of localization image acquisition, verification, and interaction shown in FIG. 3. As shown in FIG. 3, after user manual localization or automatic localization operation, a localization image is obtained, and then localization verification is performed on the localization image. A target of the localization verification is an object within the selection result (localization box) selected automatically or manually. Therefore, the task of the localization verification may be considered as simultaneously checking whether the following two dimensions meet the requirements: (1) Whether the localization image is suitable; (2) Whether the correct localization (box) is marked. When the result of the localization verification is that the localization image meets the first condition, subsequent operations are continued. When the result of the localization verification is that the localization image partially does not meet the requirements, but the user, after self-checking the localization image, considers the quality acceptable, an acceptance instruction is triggered (i.e., the deviation degree exceeds the first threshold but the user's acceptance instruction is received), and subsequent operations are continued. When the result of the localization verification is that the localization image completely does not meet the requirements, i.e., the deviation degree exceeds the first threshold, or, the deviation degree exceeds the first threshold, but no user acceptance instruction is received, the first prompt information is displayed, automatically triggering re-localization.

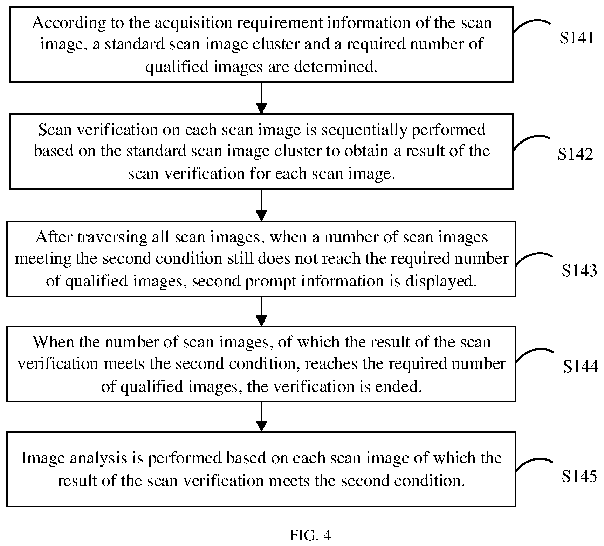

In some embodiments, as shown in FIG. 4, the operation S140 of, according to the requirements for acquiring the scan image, performing a scan verification on the scan image, and displaying second prompt information if a result of the scan verification does not meet a second condition, includes operations at blocks as follows.

At block S141: According to the acquisition requirement information of the scan image, a standard scan image cluster and a required number of qualified images are determined.

At block S142: Scan verification on each scan image is sequentially performed based on the standard scan image cluster to obtain a result of the scan verification for each scan image.

At block S143: After traversing all scan images, when a number of scan images meeting the second condition still does not reach the required number of qualified images, second prompt information is displayed.

At block S144: When the number of scan images, of which the result of the scan verification meets the second condition, reaches the required number of qualified images, the verification is ended.

At block S145: An image analysis is performed based on each scan image of which the result of the scan verification meets the second condition.

It is understandable that different requirements for acquiring scan images for subsequent processing correspond to different verification requirements for the scan image. Therefore, when verifying the scan image after obtaining it, the standard scan image cluster may be determined according to the acquisition requirement information of the scan image. In addition, for different parts and orientation types, the scan image characteristics may also be different. Therefore, the standard scan image cluster may be jointly determined by combining the acquisition requirement information of the scan image and the type and orientation of the to-be-scanned object. Similar to the construction of the standard localization image cluster, these standard scan image clusters may also be pre-built. For example, images of relevant parts scanned under correct operations of healthy subjects may be collected to obtain several scan images with different orientation types and good imaging quality, forming a standard scan image cluster. In some embodiments, standard scan images are not limited to images of a single subject; scan images from multiple different subjects may be collected to reduce the impact of individual differences on subsequent result of the scan verifications and improve the reliability of result of the scan verifications. After the current scan image acquisition is completed, its similarity with each standard scan image in the standard scan image cluster is calculated to determine the result of the scan verification. For example, a second feature extraction model may be adopted to perform feature extraction on the scan image and each standard scan image respectively, and then the similarity between the feature vector of the scan image and the feature vectors of each standard scan image may be obtained. The similarity calculation methods may be vector distance measurement methods such as Euclidean distance, Manhattan distance, cosine distance, Mahalanobis distance, and their variants. The architecture of the second feature extraction model may be the same as or different from that of the first feature extraction model. Both may be any encoder structure, including convolutional neural networks, recurrent neural networks, Vision Transformer (ViT) and its variants. The training data and weight parameters of the two feature extraction models may be the same or different. The two networks may be pre-trained together or trained separately. The relevant description for the first feature extraction model also applies to the second feature extraction model and will not be repeated here.

In some embodiments, the evaluation criteria for the "good imaging quality" typical images selected to generate the standard scan image cluster may combine subjective and objective assessments, which may be based on one or more of the following guidelines.
1. The image contrast and signal-to-noise ratio of a scan image meet general quality requirements for that type of diagnostic/scan image or parameter image.
2. There are no significant artifacts that affect reading the scan image or diagnosis.
3. The scanning direction of the scan image conforms to preset requirements for a corresponding scanning sequence (for example, cardiac diagnostic images require a short-axis orientation, and MRCP requires a coronal orientation, etc.).
4. The tissue, organ, or area of interest (to-be-scanned object) in the scan image is displayed completely and clearly.

In addition, depending on the acquisition requirement information of the scan image, the required number of qualified images may be any positive integer from 1 to N (where N is the total number of scanned images acquired). For example, acquiring scan images for TI time calculation requires at least one qualified image. As another example, cardiac function analysis requires multiple images. In some embodiments, the required number of qualified images is determined according to the requirements for acquiring the scan image. During scan verification, scan verification is performed sequentially on each scan image based on the standard scan image cluster to obtain the result of the scan verification for each scan image. After traversing all scan images, when the number of scan images meeting the second condition still does not reach the required number of qualified images, the second prompt information is displayed to prompt the user to reacquire scan images. When, during the verification process, the number of scan images whose result of the scan verification meets the second condition has reached the required number of qualified images, the verification may be ended, and subsequent image analysis may be performed based on each scan image whose result of the scan verification meets the second condition.

In other embodiments, the feature vectors of each standard scan image may be extracted in advance via the second feature extraction model, and these feature vectors are saved as a standard scan vector cluster. After acquiring the scan image, only the feature vector of the scan image is required to be extracted, and then similarity is directly calculated with the feature vectors of each standard scan image.

It is understandable that the standard scan image cluster may be updated from time to time. Users may independently add custom standard scan images or delete existing standard scan images that do not meet their requirements, making the verification of scan images more adaptable to the user's scanning habits.

In some embodiments, before performing the scan verification on the obtained scan image, the scan image may be preprocessed to improve its quality. The preprocessing method may be similar to the preprocessing method for the localization image mentioned earlier and are not repeated here.

In the embodiments, by verifying the acquired scan images one by one, it may be ensured that only images meeting specific standards, which may be reflected by the acquisition requirement information of the scan image, are selected. This precise verification may exclude low-quality or unqualified images, thereby improving the reliability and effectiveness of the final analysis results.

In some embodiments, similar to the aforementioned semantic quality control method for localization images, the present disclosure further provides a semantic quality control method for scan images, which will not be detailed again here.

To guide the model for professional quality control, a preset prompt template may be adopted. For example, for scan image quality control, the prompt may be designed as: "You are a senior MRI imaging technician, checking the quality of a diagnostic/scanning image. Examination sequence: {sequence_type}, Diagnostic purpose: {diagnostic_purpose}. Please analyze the following aspects and output in a structured format: 1. Image quality score; 2. Signal-to-noise ratio assessment; 3. Contrast assessment; 4. Artifact detection; 5. Whether the image meets subsequent diagnosis/analysis requirements; 6. If not, the problem and suggestions".

In some embodiments, the second condition includes that a deviation degree of the scan image relative to the standard scan image cluster does not exceed a second threshold, or, the deviation degree exceeds the second threshold but a user's acceptance instruction is received. The method further includes: when the result of the scan verification does not meet the second condition, displaying the deviation degree of the scan image relative to the standard scan image cluster, and/or a heat map of the scan image, and taking the deviation degree and/or the heat map as the second prompt information; where the heat map is configured to indicate an abnormality degree of the scan image.

In some embodiments, when the result of the scan verification does not meet the second condition, meaning it does not meet the requirements for subsequent analysis, the deviation degree of the scan image relative to the standard scan image cluster, and/or, the heat map of the scan image configured to indicate the abnormality degree may be displayed as the second prompt information. Specifically, displaying the deviation degree may be shown while prompting that the scan image does not meet requirements. Specifically, the difference between an average similarity between the current scan image and each standard scan image and the second threshold may be displayed. When the average value is only slightly greater than the second threshold, the user may be prompted to manually intervene and check (e.g., the user decides whether to proceed to the next step). When the average value is far greater than the second threshold, the user may be prompted, and the previous operations may be repeated directly without requesting user permission. For the heat map, an anomaly score may be calculated through similarity and threshold comparation to determine the severity of the anomaly and the handling method (i.e., user prompting, re-scanning, etc.). While prompting that the requirements are not met, the feature vector is mapped back to the image size, and the heat map indicates the abnormality degree of the corresponding area.

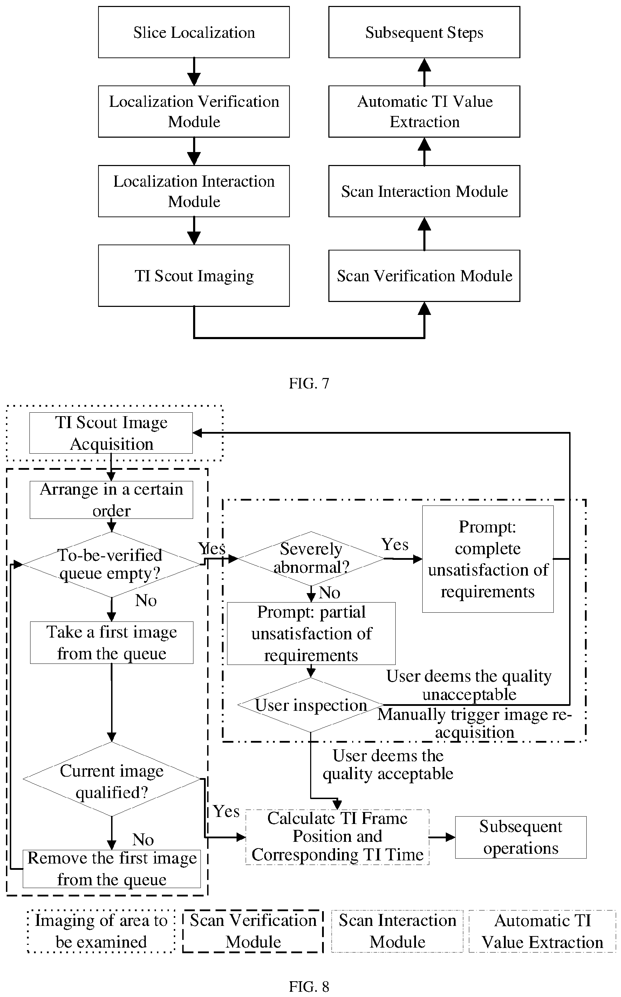

In some embodiments, the user's determination criteria are related to actual requirements. The quality verification in the scanning phase is generally associated with subsequent actions. As described in the embodiments shown in FIG. 7 regarding automatic TI time calculation based on TI Scout image quality verification, the TI Scout images acceptable to the algorithm for TI time calculation are required to meet the following quality conditions:
1. The TI Scout scan orientation/direction is cardiac short-axis, two-chamber, or four-chamber view.
2. The image has no obvious artifacts affecting myocardial visualization.

When the algorithm determines that the above quality conditions are not met, the user may decide to accept the TI Scout image quality based on the actual presentation of the image, judging that the deviation in the scan direction has not altered the anatomical morphology of the short-axis/two-chamber/four-chamber image, or that the artifact has not reduced the myocardial display effectiveness.

In other words, regarding the embodiments of user acceptance scenarios in terms of the "first condition" and the "second condition", both the "first condition" and the "second condition" include two scenarios: first, the image quality fully meets the standard (deviation degree does not exceed the threshold); second, the quality, while not fully meeting the standard (deviation degree exceeds the threshold), is accepted by the user based on clinical practical judgment. This design may provide the system with necessary flexibility, avoid unnecessary re-scans caused by overly stringent algorithms, and thus enhance the efficiency and user experience of the scanning workflow.

In some embodiments, a new second threshold may be determined in response to a user's modification operation, making the new second threshold more consistent with user habits.

As in the embodiments of localization image verification, the general deviation degree of an image relative to a standard image includes deviations in three dimensions: modality, semantics, and pathology. For the verification of the scan image in the embodiments, the main goal of verification is to ensure that there are no serious quality issues in the target area, while pathological issues may not be considered for now. Therefore, the baseline for verifying the scan image may consider only the image semantics. Therefore, the second threshold may be set to a value less than a semantic deviation value and greater than a pathological deviation value, such that during verification, there are only two cases: "with semantic deviation" and "without semantic deviation". Correspondingly, the deviation degree of the scan image relative to the standard scan image cluster is the deviation degree in image semantics. The semantic deviation value may be obtained by calculating an average distance of historical scan images with semantic deviation relative to the standard scan image cluster. The pathological deviation value may be obtained by calculating an average distance of historical scan images with pathological deviation relative to the standard scan image cluster.

In some embodiments, the second condition is set as the deviation degree of the scan image from the standard scan image cluster not exceeding the second threshold, to ensure scanning accuracy. In addition, it is set that even if the deviation degree exceeds the second threshold, the user is still allowed to choose to accept it under the premise of fully understanding the risks. This flexibility may adapt to different clinical requirements, avoiding unnecessary repeated scans due to overly strict judgment standards, thereby improving scanning efficiency. In some embodiments, considering that during scan verification, scenarios that are more focused on local area abnormalities, the heat map is adopted instead of warning as the second prompt information to improve the effectiveness and accuracy of the prompt information.

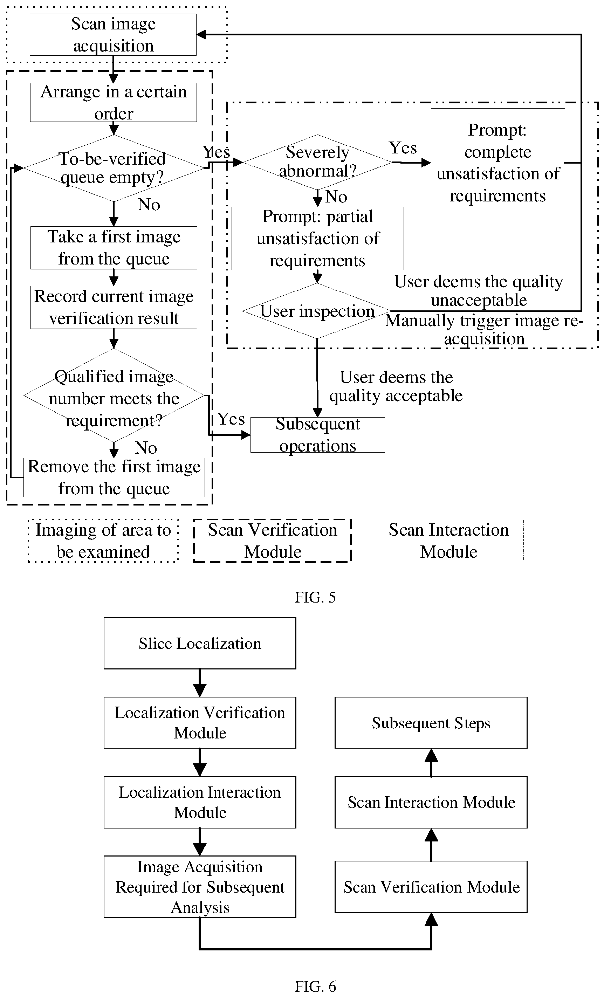

To facilitate understanding of the acquisition, verification, and interaction of the scan image in the above embodiment, further explanation is provided below in conjunction with the schematic flowchart of scan image acquisition, verification, and interaction shown in FIG. 5.

As shown in FIG. 5, after acquiring scan images, the scan images are first arranged in a certain order (e.g., a time order) to form a to-be-verified queue. Each time, one scan image is taken from the to-be-verified queue for verification, and the deviation degree of this scan image relative to the standard scan image cluster is determined. When the deviation degree does not exceed the second threshold, the corresponding scan image is considered qualified; otherwise (when the deviation degree exceeds the second threshold), the corresponding scan image is unqualified. After each verification, the number of qualified images is updated. When the obtained number of qualified images does not meet the requirement, a next scan image is taken for verification until the number of qualified images meets the requirement, then verification is ended, and subsequent operations are executed. The sequence described may be based on scan time. In some embodiments, the deviation degree may be an average among deviation degrees of this scan image relative to images included in the standard scan image cluster.

When, after traversing all scan images in the to-be-verified queue, the number of scan images meeting the condition that the deviation degree does not exceed the second threshold still does not reach the required number of qualified images, then recall may be performed from the scan images whose deviation degree exceeds the second threshold. The deviation degree of each scan image relative to the standard scan image cluster is determined, i.e., whether it is severely abnormal. When the scan image is severely abnormal, meaning the scan is completely unqualified, rescanning may be automatically triggered. When scan image is only partially unqualified, i.e., the deviation degree does not exceed the second threshold, the user may decide whether to recall the scan image after self-checking the scan image. When the user considers the scan quality acceptable, the scan image may be recalled for participation in subsequent operations. When the user considers the scan quality unacceptable, rescanning may be automatically triggered.

It should be noted that image acquisition and quality control in FIG. 5 are executed sequentially, i.e., scan verification is performed after all scan images are acquired. In practical applications, the two may be asynchronous, i.e., verification may start as long as at least one scan image to be verified is acquired.

In some embodiments, to more clearly illustrate the embodiments of the present disclosure, the following description will be combined with specific examples in the accompanying drawings. Referring to FIG. 6, a schematic flowchart of a medical imaging scanning method is shown. First, slice localization is performed. After localization is completed, the localization image is verified by a localization verification module, and a localization interaction module performs localization interaction (e.g., displaying first prompt information) according to the result of the localization verification. After the localization verification passes, scan image acquisition required for subsequent analysis is performed. The scan image is verified by a scan verification module, and a scan interaction module performs scan interaction (e.g., displaying second prompt information) according to the result of the scan verification. Finally, after the scan verification passes, subsequent operations are executed.

In some embodiments, taking MR cervical spine scanning as an example, the quality control verification in the localization phase and the scanning phase is as follows.
1. Slice Localization: Automatic/manual positioning is performed based on a neck localization image (spine_scout) to obtain a localization result/ The localization result includes the sagittal MPR (MultiPlanar Reconstruction) and directional localization results for each intervertebral disc.
2. Localization Verification Module: For the sagittal MPR and each intervertebral disc slice image, a neural network is used for quality verification respectively. For the sagittal MPR, poor quality typically manifests as incomplete display of intervertebral discs and vertebral bodies. For the intervertebral disc slice images, poor quality generally appears as incomplete display of the intervertebral disc. When the localization verification module reports an abnormal quality control result, the user may inspect the localization result to decide whether to accept the corresponding image. When the user does not accept, the user may re-scan the localization image or perform manual slice localization.
3. Image Acquisition for Subsequent Analysis (Scanning Image Acquisition): The cervical T1/T2 sequence images are scanned.
4. Scanning Verification Module: Quality verification is performed on the T1/T2 sequence images. For example, when the T2 image exhibits poor fat suppression or severe artifacts, the quality verification module may determine the quality is poor and feedback to the user, and the user may then decide on actions such as re-scanning or changing a scanning scheme (e.g. scanning direction/orientation).

Referring to FIG. 7, FIG. 7 is a schematic diagram of a workflow for automatically calculating Inversion Time (TI time) according to some embodiments of the present disclosure. During the magnetic resonance scanning process, before scanning a sequence for cardiac delayed enhancement, an Inversion Time scout (TI Scout) sequence is required to be scanned. This sequence acquires multiple images of the same Field of View (FOV) within a certain period of time. The user observes the TI Scout images and manually enters the TI value corresponding to the darkest myocardial image into the subsequent delayed enhancement sequence, such as the Phase-Sensitive Inversion Recovery (PSIR) sequence or Inversion Recovery (IR) sequence, etc., to obtain images with better contrast. Using the method of the present disclosure may automatically detect the TI value, thereby optimizing the user experience, reducing the application difficulty of this sequence, and improving the scanning success rate. For image verification, as long as at least one TI Scout image meets the requirements of the subsequent TI value automatic extraction algorithm, it is sufficient. Referring to and simplifying FIG. 5, the quality control and interaction process for TI Scout images may be simplified as shown in FIG. 8.

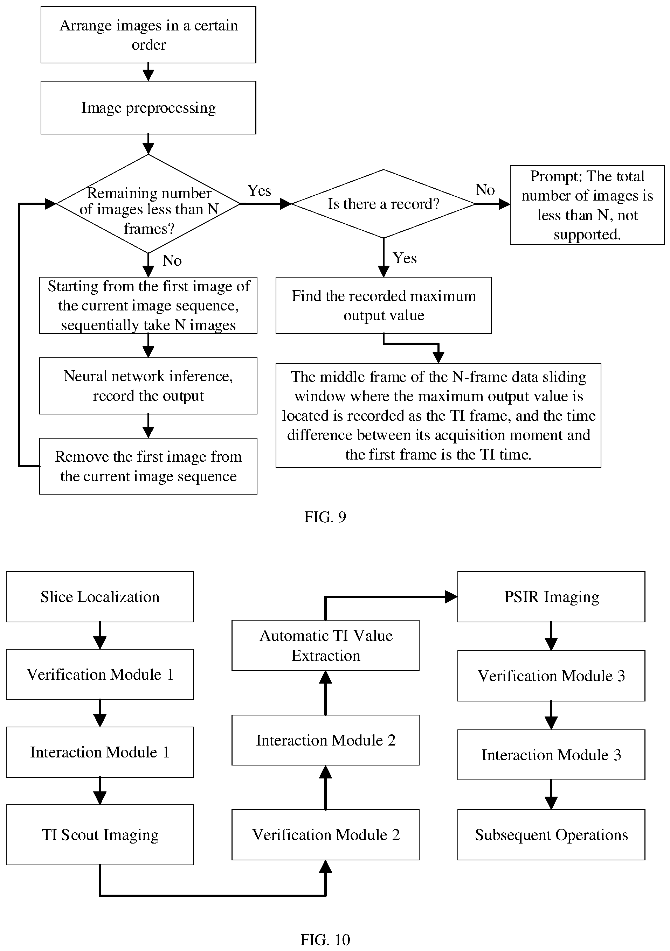

For automatically identifying the TI value, deep learning networks may be adopted to directly predict cardiac TI frames and automatically obtain the TI value. The choice of deep learning networks may include: detection networks, segmentation networks, localization networks, networks combining multiple networks, or multi-task combined networks, etc. When a multi-network combination is adopted, for example, a combination of a detection network and a recurrent network may be used (the detection network may be MobileNet, a lightweight deep learning convolutional neural network; the recurrent network may be Long Short-Term Memory network (LSTM); the network combination may be called a TI Scout network). The TI Scout network outputs a probability value. Data is input into the network as a sliding window every N frames (where N is a prime number, e.g., 5). The output is the probability that the sliding window is a TI frame. The middle frame of the N-frame data sliding window where the maximum probability value index is located is the TI frame. This series of processes is shown in FIG. 9.

In some embodiments (referring to FIG. 9), the term "record" refers to a process where, after the image is input into the neural network, the network generates and outputs a predicted probability value. This probability value is configured to indicate the likelihood of an image (or a specific frame within an image sequence) being a "TI frame". Ultimately, the inversion time (TI) may be determined by selecting the frame with the highest probability.

In the schematic diagram shown in FIG. 7, the purpose of calculating TI time is usually for subsequent PSIR imaging. Therefore, a fully automated PSIR workflow with full-process quality control may be further considered. Due to the application of modules such as automatic localization, automatic quality control, and automatic TI frame calculation, the entire process does not require active intervention, as shown in FIG. 10. In addition, besides the method shown in FIG. 7-FIG. 9, the TI Scout value may also be considered to be obtained directly by a single network. For example, only a detection network or a recurrent neural network is adopted to directly regress the probability value and judge the TI frame. When a multi-task network is adopted, such as Region-based Convolutional Neural Network (Mask R-CNN), the myocardium may be segmented first, and then the probability value is regressed for the myocardial region to judge the TI frame.

It is understandable that a verification module 1 and an interaction module 1 in the application example in FIG. 10 may correspond to the localization verification module and localization interaction module in the embodiments of FIG. 6; a verification module 2 and an interaction module 2 may correspond to the scan verification module and scan interaction module in the embodiments of FIG. 6. The construction and operation process of the newly added verification module 3 and interaction module 3 may refer to the two verification modules and interaction modules in FIG. 6, with the purpose of ensuring the quality of PSIR images. Thus, it may be seen that using the method of the present disclosure, verification and interaction modules may be flexibly combined according to actual requirements. The entire workflow does not require active intervention, which may greatly improve efficiency. Moreover, in terms of detection and interaction, the detection module and interaction module may be inserted at any position of any medical imaging workflow without modifying upstream and downstream task modules, and may be user-customized according to task requirements and on-site actual conditions.

It should be understood that although the operations in the flowcharts involved in the above-mentioned embodiments are shown sequentially according to the direction of the arrows, these operations are not necessarily executed in the order indicated by the arrows. Unless explicitly stated herein, the execution of these operations is not strictly order-limited, and these operations may be executed in other orders. Moreover, at least part of the operations in the flowcharts involved in the above-mentioned embodiments may include multiple operations or multiple stages. These operations or stages are not necessarily executed at the same time but may be executed at different times. The execution order of these operations or stages is not necessarily sequential but may be performed in turn or alternately with other operations or at least part of the operations or stages of other operations.

It can be understood that, based on the above embodiments, the present disclosure can be deemed to disclose a medical imaging scanning method, including:
obtaining a localization image of a to-be-scanned object after the to-be-scanned object is located at a position;
generating a localization verification result by performing a localization verification based on the localization image; and
displaying the localization verification result on an interface.

In a case where the localization verification result meets a first condition, the method further includes: performing an image scanning on the to-be-scanned object.

In a case where the localization verification result does not meet the first condition, the performing the localization verification further includes: prompting to a user for inspection; in response to a confirmation from the user indicating that the user does not accept the position of the to-be-scanned object, the method further includes: in response to a re-locating instruction initiated by the user, re-locating the to-be-scanned object according to the re-locating instruction; or in response to another confirmation from the user indicating that the user accepts the position of the to-be-scanned object, the method further includes: performing the image scanning on the to-be-scanned object.

Based on the same inventive concept, embodiments of the present disclosure further provide a medical imaging scanning system for implementing the above-mentioned medical imaging scanning method. The implementation solutions for solving problems provided by this system are similar to those recorded in the above method. Therefore, specific limitations in one or more medical imaging scanning system embodiments provided below may be referred to the limitations on the medical imaging scanning method above and are not repeated here.

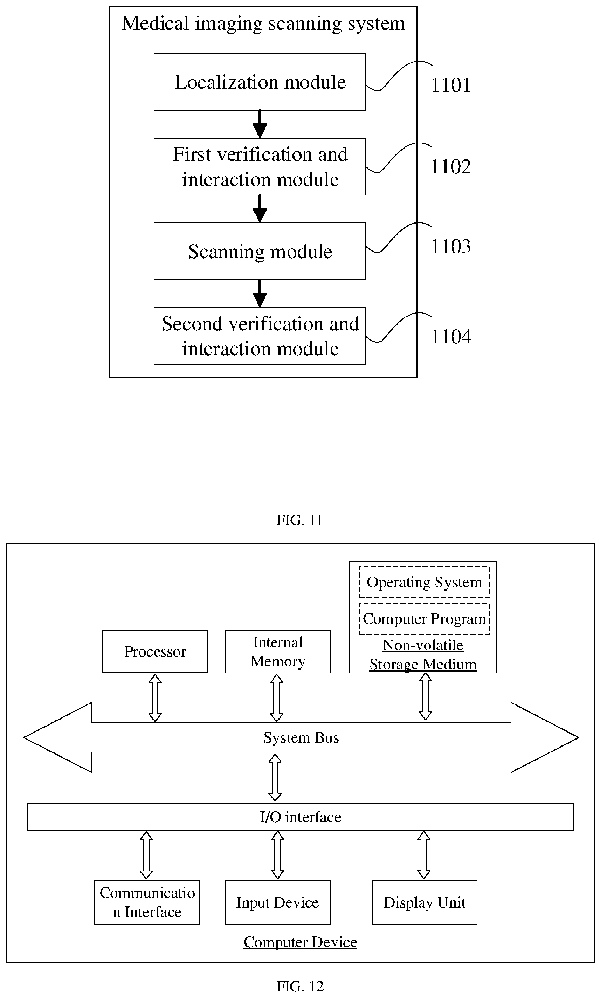

In some embodiments, as shown in FIG. 11, a medical imaging scanning system is provided, including:
a localization module 1101, configured to, in response to a localization instruction for a to-be-scanned object, acquire a localization image of the to-be-scanned object;
a first verification and interaction module 1102, configured to perform localization verification on the localization image, and display first prompt information when a result of the localization verification does not meet a first condition; where the first prompt information is configured to prompt to perform re-localization of the to-be-scanned object until the result of the localization verification meets the first condition;
a scanning module 1103, configured to, at a position corresponding to a result of the localization verification meeting the first condition, perform an image scanning on the to-be-scanned object to obtain a scan image of the to-be-scanned object; and
a second verification and interaction module 1104, configured to, according to acquisition requirement information of the scan image, perform a scan verification on the scan image, and display second prompt information when a result of the scan verification does not meet a second condition; where the second prompt information is configured to prompt rescanning the to-be-scanned object until a scan image meeting the second condition is obtained.

In some embodiments, the first verification and interaction module 1102 is further configured to determine a standard localization image cluster based on a type of the to-be-scanned object, where the type includes an orientation corresponding to the localization instruction; and verify the localization image based on the standard localization image cluster to obtain the result of the localization verification.

In some embodiments, the first condition includes that a deviation degree of the localization image relative to the standard localization image cluster does not exceed a first threshold, or, the deviation degree exceeds the first threshold but a user's acceptance instruction is received; the first verification and interaction module 1102 is further configured to, when the result of the localization verification does not meet the first condition, display warning information, and/or the deviation degree of the localization image relative to the standard localization image cluster, and take the displayed warning information and/or the deviation degree as the first prompt information.

In some embodiments, the deviation degree of the localization image relative to the standard localization image cluster is a deviation degree in an image modality; the first threshold is a value less than a modality deviation value and greater than a semantic deviation value; the modality deviation value is obtained by calculating an average distance of historical localization images with modality deviation relative to the standard localization image cluster, and the semantic deviation value is obtained by calculating an average distance of historical localization images with semantic deviation relative to the standard localization image cluster.

In some embodiments, there are multiple scan images; the second verification and interaction module 1104 is further configured to: determine a standard scan image cluster and a required number of qualified images according to the acquisition requirement information of the scan image; sequentially perform scan verification on each scan image based on the standard scan image cluster to obtain a result of the scan verification for each scan image; and after traversing all scan images, when the number of scan images meeting the second condition still does not reach the required number of qualified images, display the second prompt information.

In some embodiments, the second verification and interaction module 1104 is further configured to: when the number of scan images, of which the result of the scan verification meets the second condition, reaches the required number of qualified images, end the verification; and perform image analysis based on each scan image of which the result of the scan verification meets the second condition.

In some embodiments, the second condition includes that a deviation degree of the scan image relative to the standard scan image cluster does not exceed a second threshold, or, the deviation degree exceeds the second threshold but a user's acceptance instruction is received; the second verification and interaction module 1104 is further configured to: when the result of the scan verification does not meet the second condition, display the deviation degree of the scan image relative to the standard scan image cluster, and/or a heat map of the scan image, and take the deviation degree and/or the heat map as the second prompt information; where the heat map is configured to indicate an abnormality degree of the scan image.

In some embodiments, the deviation degree of the scan image relative to the standard scan image cluster is a deviation degree in image semantics; the second threshold is a value less than a semantic deviation value and greater than a pathological deviation value; the semantic deviation value is obtained by calculating an average distance of historical scan images with semantic deviation relative to the standard scan image cluster, and the pathological deviation value is obtained by calculating an average distance of historical scan images with pathological deviation relative to the standard scan image cluster.

Each module in the above medical imaging scanning system may be implemented entirely or partially by software, hardware, and combinations thereof. The above modules may be embedded in or independent of the processor of the computer device in the form of hardware, or may be stored in the memory of the computer device in the form of software for the processor to call and execute the operations corresponding to the above modules.

In some embodiments, a computer device is provided. The computer device may be a terminal, and its internal structure diagram may be as shown in FIG. 12. The computer device includes a processor, a memory, a communication interface, a display screen, and an input device connected via a system bus. The processor of the computer device is configured to provide computing and control capabilities. The memory of the computer device includes a non-volatile storage medium and an internal memory. The non-volatile storage medium stores an operating system and a computer program. The internal memory provides an environment for the operation of the operating system and computer program in the non-volatile storage medium. The communication interface of the computer device is used for wired or wireless communication with external terminals. Wireless communication may be implemented through WIFI, mobile cellular networks, NFC (Near Field Communication), or other technologies. The computer program, when executed by the processor, implements a medical imaging scanning method. The display screen of the computer device may be a liquid crystal display or an electronic ink display screen. The input device of the computer device may be a touch layer covering the display screen, buttons, a trackball, or a touchpad set on the computer device housing, or may be an external keyboard, touchpad, or mouse, etc.

Those skilled in the art can understand that the structure shown in FIG. 12 is only a block diagram of a part of the structure related to the solution of the present disclosure and does not constitute a limitation on the computer device to which the solution of the present disclosure is applied. A specific computer device may include more or fewer components than shown in the figure, or combine some components, or have a different arrangement of components.

In some embodiments, a computer device is further provided, including a memory and a processor. The memory stores a computer program, and when the processor executes the computer program, the operations in the above method embodiments are implemented.

In some embodiments, a computer-readable storage medium is provided, having a computer program stored thereon. When the computer program is executed by a processor, the operations in the above method embodiments are implemented.

In some embodiments, a computer program product is provided, including a computer program. When the computer program is executed by a processor, the operations in the above method embodiments are implemented.

It should be noted that the user information (including but not limited to user device information, user personal information, etc.) and data (including but not limited to data used for analysis, stored data, displayed data, etc.) involved in the present disclosure are all authorized by users or fully authorized by relevant parties, and the collection, use, and processing of related data need to comply with relevant laws, regulations, and standards of relevant countries and regions.

Those of ordinary skill in the art can understand that all or part of the processes for implementing the methods in the above embodiments may be accomplished by instructing relevant hardware through a computer program. The computer program may be stored in a non-volatile computer-readable storage medium. When the computer program is executed, it may include the processes of the embodiments of the above methods. Any reference to memory, database, or other media in the embodiments provided in the present disclosure may include at least one of non-volatile and volatile memory. Non-volatile memory may include read-only memory (ROM), magnetic tape, floppy disk, flash memory, optical memory, high-density embedded non-volatile memory, resistive memory (ReRAM), magnetoresistive random access memory (MRAM), ferroelectric memory (FRAM), phase change memory (PCM), graphene memory, etc. Volatile memory may include random access memory (RAM) or external cache memory, etc. By way of illustration and not limitation, RAM may be in various forms, such as static random access memory (SRAM) or dynamic random access memory (DRAM), etc. The database involved in the embodiments provided in the present disclosure may include at least one of relational databases and non-relational databases. Non-relational databases may include blockchain-based distributed databases, etc., and are not limited thereto. The processor involved in the embodiments provided in the present disclosure may be a general-purpose processor, a central processing unit, a graphics processing unit, a digital signal processor, a programmable logic device, a data processing logic based on quantum computing, etc., and is not limited thereto.

The technical features of the above embodiments may be combined arbitrarily. For the sake of brevity, not all possible combinations of the technical features in the above embodiments are described. However, as long as there is no contradiction in the combination of these technical features, they should be considered as within the scope of this specification.

## Claims

1. A medical imaging scanning method, **characterized by** comprising:
in response to a localization instruction for a to-be-scanned object, acquiring (S110) a localization image of the to-be-scanned object;
performing (S120) a localization verification on the localization image, and in a case where a result of the localization verification fails to meet a first condition, displaying (S120) first prompt information; wherein the first prompt information is configured to prompt to perform re-localization of the to-be-scanned object until the result of the localization verification meets the first condition;
at a position corresponding to a result of the localization verification meeting the first condition, performing (S130) an image scanning on the to-be-scanned object to obtain at least one scan image of the to-be-scanned object; and
according to acquisition requirement information of the at least one scan image, performing (S140) a scan verification on the at least one scan image; and in response to a result of the scan verification failing to meet a second condition, displaying (S140) second prompt information; wherein the second prompt information is configured to prompt rescanning the to-be-scanned object until obtaining at least one new scan image meeting the second condition.

2. The method according to claim 1, wherein the localization instruction is an orientation determination instruction for the to-be-scanned object during scanning; the performing (S120) a localization verification on the localization image comprises:
determining a standard localization image cluster according to a type of the to-be-scanned object; wherein the type of the to-be-scanned object comprises an orientation corresponding to the localization instruction or a body part corresponding to the to-be-scanned object; and
verifying the localization image based on the standard localization image cluster to obtain the result of the localization verification.

3. The method according to claim 2, wherein the first condition comprises that a deviation degree of the localization image relative to the standard localization image cluster does not exceed a first threshold, or, the deviation degree exceeds the first threshold but a user acceptance instruction is received; the method further comprises:
in response to the result of the localization verification failing to meet the first condition, displaying at least one of warning information or the deviation degree of the localization image relative to the standard localization image cluster; and
taking the at least one of the warning information or the deviation degree as the first prompt information.

4. The method according to claim 3, wherein the deviation degree of the localization image relative to the standard localization image cluster is a deviation degree in an image modality;
the first threshold is a value less than a modality deviation value and greater than a semantic deviation value; wherein the modality deviation value is obtained by calculating an average distance of historical localization images with modality deviation relative to the standard localization image cluster, and the semantic deviation value is obtained by calculating an average distance of historical localization images with semantic deviation relative to the standard localization image cluster.

5. The method according to any one of claims 1-4, wherein the at least one scan image is a plurality of scan images;
the performing (S140) a scan verification on the at least one scan image, and the displaying second prompt information comprise:
determining (S141) a standard scan image cluster and a required number of qualified images according to the acquisition requirement information;
sequentially performing (S142) a scan verification on each of the plurality of scan images based on the standard scan image cluster to obtain the result of the scan verification for each scan image; and
in response to, after traversing all the plurality of scan images, a number of scan images, of which the result of the scan verification meets the second condition, still not reaching the required number of qualified images, displaying (S143) the second prompt information.

6. The method according to claim 5, further comprising:
in response to the number of scan images, of which the result of the scan verification meets the second condition, reaching the required number of qualified images, ending (S144) the scan verification; and
performing (S145) an image analysis according to each of the scan images, of which the result of the scan verification meets the second condition.

7. The method according to claim 5 or 6, wherein for each of the plurality of scan images, the second condition comprises that a deviation degree of the scan image relative to the standard scan image cluster does not exceed a second threshold, or, the deviation degree exceeds the second threshold but a user acceptance instruction is received; the method further comprises:
in response to the result of the scan verification of the scan image failing to meet the second condition, displaying at least one of the deviation degree of the scan image relative to the standard scan image cluster or a heat map of the scan image; and
taking the at least one of the deviation degree or the heat map as the second prompt information; wherein the heat map is configured to indicate an abnormality degree of the scan image.

8. The method according to claim 7, wherein the deviation degree of the scan image relative to the standard scan image cluster is a deviation degree in image semantics;
the second threshold is a value less than a semantic deviation value and greater than a pathological deviation value; wherein the semantic deviation value is obtained by calculating an average distance of historical scan images with semantic deviation relative to the standard scan image cluster, and the pathological deviation value is obtained by calculating an average distance of historical scan images with pathological deviation relative to the standard scan image cluster.

9. The method according to any one of claims 1-8, wherein the localization verification is performed via a visual language model and the performing the localization verification on the localization image comprises:
obtaining input information, wherein the input information includes the localization image, contextual information, or one or more preset prompt templates; and
performing the localization verification by processing the input information by the visual language model.

10. The method according to any one of claims 1-9, wherein the scan verification is performed via a visual language model, and the performing the scan verification on the at least one scan image comprises:
obtaining input information, wherein the input information includes the at least one scan image, contextual information, or one or more preset prompt templates; and
performing the scan verification by processing the input information by the visual language model.

11. The method according to any one of claims 1-10, wherein the first condition comprises that an image quality of the localization image meets a clinical demand and the to-be-scanned object is within a field of view (FOV) of a medical imaging equipment.

12. The method according to any one of claims 1-11, wherein the second condition comprises that an image quality of the at least one scan image meets a clinical demand for subsequent medical analysis and diagnosis.

13. A medical imaging scanning system, **characterized by** comprising:
a localization module (1101), configured to, in response to a localization instruction for a to-be-scanned object, acquire a localization image of the to-be-scanned object;
a first verification and interaction module (1102), configured to perform a localization verification on the localization image, and display first prompt information in a case where a result of the localization verification does not meet a first condition; wherein the first prompt information is configured to prompt to perform re-localization of the to-be-scanned object until the result of the localization verification meets the first condition;
a scanning module (1103), configured to, at a position corresponding to a result of the localization verification meeting the first condition, perform an image scanning on the to-be-scanned object to obtain at least one scan image of the to-be-scanned object; and
a second verification and interaction module (1104), configured to, according to acquisition requirement information of the at least one scan image, perform a scan verification on the at least one scan image, and display second prompt information in response to a result of the scan verification failing to meet a second condition; wherein the second prompt information is configured to prompt rescanning the to-be-scanned object until obtaining at least one new scan image meeting the second condition.

14. A computer device, comprising a memory and a processor; wherein the memory stores a computer program; the processor, when executing the computer program, implements the medical imaging scanning method according to any one of claims 1 to 12.

15. A computer-readable storage medium, storing a computer program; wherein, when the computer program is executed by a processor, the processor is caused to perform the medical imaging scanning method according to any one of claims 1 to 12.
